# FASCICULE DE BREVET EUROPEEN

(11) **EP 1 742 644 B1**
(45) Date de publication et mention de la délivrance du brevet: **28.12.2011**
(21) Numéro de dépôt: 05762344.9
(22) Date de dépôt: 26.04.2005
(51) Int. Cl.: A61K 31/7076, A61K 31/5513, A61P 35/00

(54) **COMPOSITION THÉRAPEUTIQUE CONTENANT AU MOINS UN DÉRIVÉ DE LA PYRROLOBENZODIAZÉPINE ET LA FLUDARABINE**
THERAPEUTISCHE ZUSAMMENSETZUNGEN MIT MINDESTENS EINEM PYRROLOBENZODIAZEPIN-DERIVAT UND FLUDARABIN
THERAPEUTIC COMPOSITIONS CONTAINING AT LEAST ONE PYRROLOBENZODIAZEPINE DERIVATIVE AND FLUDARABINE

(30) Priorité: 27.04.2004 FR 0404424
(43) Date de publication de la demande: 17.01.2007
(73) Titulaire: IPSEN PHARMA, 92100 Boulogne-Billancourt (FR)
(72) Inventeur: DELAVAULT, Patrick, F-92700 Colombes (FR); PEPPER, Chris, Vale of Glamorgan CF64 5QD (GB)
(74) Mandataire: Watson, Robert James
(86) Numéro de dépôt international: PCT/FR2005/001025
(87) Numéro de publication internationale: WO 2005/105113

(56) Documents cités:
- WO-A-00/12508
- GREGSON S J ET AL: "Synthesis of a novel C2/C2'-exo unsaturated pyrrolobenzodiazepine cross-linking agent with remarkable DNA binding affinity and cytotoxicity" JOURNAL OF THE CHEMICAL SOCIETY, CHEMICAL COMMUNICATIONS, CHEMICAL SOCIETY. LETCHWORTH, GB, 1999, pages 797-798, XP002135055 ISSN: 0022-4936
- WILKINSON, GARY P. [REPRINT AUTHOR] ET AL: "Pharmacokinetics and intracellular pharmacological characteristics of the novel pyrrolobenzodiazepine (PBD) dimer SJG - 136 ." PROCEEDINGS OF THE AMERICAN ASSOCIATION FOR CANCER RESEARCH ANNUAL MEETING, (JULY 2003) VOL. 44, PP. 320. PRINT. MEETING INFO.: 94TH ANNUAL MEETING OF THE AMERICAN ASSOCIATION FOR CANCER RESEARCH. WASHINGTON, DC, USA. JULY 11-14, 2003. ISSN: 0197-016, juillet 2003 (2003-07), XP008038560
- ALLEY, MICHAEL C. [REPRINT AUTHOR] ET AL: "Efficacy evaluations of SJG - 136 (NSC 694501), a novel pyrrolobenzodiazepine dimer with broad spectrum antitumor activity." PROCEEDINGS OF THE AMERICAN ASSOCIATION FOR CANCER RESEARCH ANNUAL MEETING, (MARCH, 2002) VOL. 43, PP. 63. PRINT. MEETING INFO.: 93RD ANNUAL MEETING OF THE AMERICAN ASSOCIATION FOR CANCER RESEARCH. SAN FRANCISCO, CALIFORNIA, USA. APRIL 06-10, 2002. I, 6 octobre 2002 (2002-10-06), XP001182944
- HARTLEY, JOHN A. [REPRINT AUTHOR] ET AL: "In vitro antitumor activity and in vivo DNA interstrand crosslinking by the novel pyrrolobenzodiazepine dimer SJG - 136 (NSC 694501)." PROCEEDINGS OF THE AMERICAN ASSOCIATION FOR CANCER RESEARCH ANNUAL MEETING, (MARCH, 2002) VOL. 43, PP. 489. PRINT. MEETING INFO.: 93RD ANNUAL MEETING OF THE AMERICAN ASSOCIATION FOR CANCER RESEARCH. SAN FRANCISCO, CALIFORNIA, USA. APRIL 06-10, 2002., 3 octobre 2002 (2002-10-03), XP001182945
- CLINGEN, PETER H. [REPRINT AUTHOR] ET AL: "The role of nucleotide excision repair and homologous recombination in the sensitivity of mammalian cells to the minor groove crosslinking pyrrolo(2,1-c)(1,4)benzodiazepine dimer SJG - 136 (NSC694501)." PROCEEDINGS OF THE AMERICAN ASSOCIATION FOR CANCER RESEARCH ANNUAL MEETING, (JULY 2003) VOL. 44, PP. 524. PRINT. MEETING INFO.: 94TH ANNUAL MEETING OF THE AMERICAN ASSOCIATION FOR CANCER RESEARCH. WASHINGTON, DC, USA. JULY 11-14, 2003. ISSN: 0197-016, juillet 2003 (2003-07), XP001182946
- WILKINSON, GARY P. [REPRINT AUTHOR] ET AL: "Pharmacokinetics, metabolism & glutathione reactivity of SJG - 136 ." BRITISH JOURNAL OF CANCER, (JULY 2003) VOL. 88, NO. SUPPLEMENT 1, PP. S29. PRINT. MEETING INFO.: BRITISH CANCER RESEARCH MEETING 2003. BOURNEMOUTH, UK. JULY 02-05, 2003. ISSN: 0007-0920 (ISSN PRINT)., juillet 2003 (2003-07), XP001182947
- KAMAL A. ET AL: "Recent developments in the design, synthesis and structure-activity relationship studies of pyrrolo[2,1-c][1,4]benzodiazepines as DNA-interactive antitumour antibiotics." CURRENT MEDICINAL CHEMISTRY - ANTI-CANCER AGENTS, 2/2 (215-254). REFS: 109 ISSN: 1568-0118 CODEN: CMCACI, 2002, XP008038559
- KEATING M J ET AL: "CLINICAL EXPERIENCE WITH FLUDARABINE IN HEMATO-ONCOLOGY" HEMATOLOGY AND CELL THERAPY, SPRINGER VERLAG FRANCE, PARIS,, FR, vol. SUPPL. 2, no. 38, 1996, pages S83-S91, XP008005332 ISSN: 1269-3286
- GOODMAN AND GILMAN ED - GOODMAN GILMAN A ET AL GOODMAN GILMAN A: "Pharmacological basis of therapeutics" 2001, PHARMACOLOGICAL BASIS OF THERAPEUTICS, GOODMAN GILMAN'S PHARMACOLOGICAL BASIS OF THERAPEUTICS, NEW YORK, PERGAMON PRESS, US, PAGE(S) 1415 , XP002305148 page 1415, colonne 2, alinéa 1 - alinéa 2

## Description

La présente invention concerne l'utilisation d'une composition thérapeutique comprenant au moins un dérivé de la pyrrolobenzodiazépine selon la revendication 1 en combinaison et en particulier en combinaison synergique avec la fludarabine pour le traitement du cancer. Une telle composition est particulièrement intéressante pour le traitement de certaines maladies hématologiques. L'invention concerne également une méthode de traitement du cancer avec le dérivé de la pyrrolobenzodiazépine selon la revendication 1 en combinaison et en particulier en combinaison synergique avec la fludarabine. Enfin la présente invention concerne un produit comprenant un dérivé de la pyrrolobenzodiazépine selon la revendication 1 et la fludarabine, en tant que produit de combinaison et en particulier en tant que combinaison synergique pour une utilisation simultanée, séparée ou étalée dans le temps, pour le traitement du cancer.

La chimiothérapie et plus particulièrement la chimiothérapie combinée reposant sur l'association d'agents possédant un mécanisme d'action différent est un principe thérapeutique établi aujourd'hui pour lutter contre le cancer. Ainsi la combinaison de différents agents anti-tumoraux peut être une façon d'augmenter l'efficacité antitumorale lorsqu'un effet synergique est révélé et/ou lorsqu'une baisse de toxicité est observée.

Dans la présente demande, l'expression "dérivé de la pyrrolobenzodiazépine" comprend le produit de formule (II) 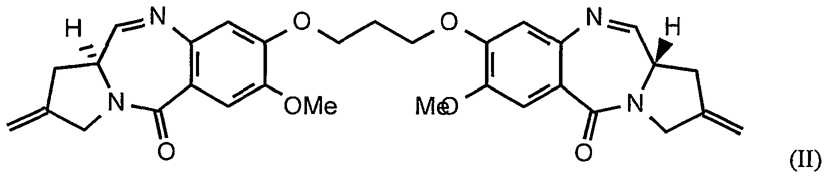 et/ou un mono- et/ou bis-carbinol formé par addition d'eau ou d'alcool,
et/ou un sel d'addition avec un acide et/ou une forme solvatée et/ou une forme hydratée,
et/ou un sel d'addition avec un acide et/ou une forme solvatée et/ou une forme hydratée d'un tel composé, sous forme amorphe ou crystalline, sous forme racémique ou sous une forme optiquement active,
et/ou tout composé qui peut générer dans des conditions physiologiques un des composés tels que définis ci-dessus,
et/ou tout mélange de quelques composés ou de tous les composés listés ci-dessus.

Par la suite, le dérivé de la pyrrolobenzodiazépine tel que défini ci-dessus, sera appelé "dérivé PBD". Un tel dérivé PBD peut être préparé selon la méthode décrite dans WO 00/12508 ou bien dans Gregson S J et al, Chem. Commun., 1999, pages 797-798. Ses propriétés ont fait l'objet de publications scientifiques (Gary P. Wilkinson, Proceedings of the American Association for Cancer Research, vol. 44, 2nd édition, July 2003 ; pp. 320 ; Michael C. Alley, Proceedings of the American Association for Cancer Research, vol. 43, March 2002, pp. 63 ; John A. Hartley, Proceedings of the American Association for Cancer Research, vol. 43, March 2002, pp. 489 ; Peter H. Clingen, Proceedings of the American Association for Cancer Research, vol. 44, 2nd edition, July 2003 ; pp. 524 ; Gary P. Wilkinson, British Journal of Cancer (2003) 88 (Suppl I), S25-54 ; A. Kamal, curr. Med. Chem. - Anti-cancer agents, 2002, 2, 215-254).

La fludarabine (commercialement disponible en France sous le nom commercial Fludara^{®}) est un produit dont les principales indications thérapeutiques sont les cancers lymphoïdes chroniques telles que leucémie lymphoïde chronique et lymphomes malins (MJ Keating, Hematol Cell Ther (1996) 38:S83-S91 ; Goodman and Gilman, Pharmacological basis of therapeutics, Pergamon Press, US, pp. 1415).

La présente invention a donc pour objet l'utilisation d'une composition thérapeutique comprenant au moins le dérivé PBD de formule (II) en combinaison avec la fludarabine, pour la préparation d'un médicament pour le traitement du cancer.

La présente invention a plus particulièrement pour objet l'utilisation telle que définie ci-dessus, pour le traitement de maladies hématologiques, préférentiellement pour le traitement de leucémies et très préférentiellement pour la leucémie lymphoïde chronique. L'invention a également pour objet une méthode de traitement du cancer, ladite méthode comprenant l'administration à un patient d'une dose thérapeutiquement ou cliniquement efficace d'une composition thérapeutique comprenant au moins un premier composé qui est le dérivé PBD de formule (II) et un second composé qui est la fludarabine. De préférence, une telle composition est administrée pour le traitement de maladies hématologiques, de manière préférentielle pour le traitement des leucémies et de manière très préférentielle pour le traitement de la leucémie lymphoïde chronique.

Enfin, la présente invention a pour objet un produit comprenant le dérivé PBD de formule (II) et la fludarabine, en tant que produit de combinaison pour une utilisation simultanée, séparée ou étalée dans le temps, pour le traitement du cancer. Plus particulièrement, un produit selon la présente invention est adapté pour traiter les maladies hématologiques, de manière préférentielle pour traiter les leucémies et de manière très préférentielle la leucémie lymphoïde chronique.

Une composition thérapeutique selon la présente invention consiste en la combinaison et en particulier une combinaison synergique du dérivé PBD de formule (II) et de la fludarabine dans laquelle le dérivé PBD de formule (II) et la fludarabine peuvent être présents sous une forme non séparée et peuvent donc être administrés simultanément, ou sous forme séparée et ainsi être administrés de façon simultanée ou séparée ou étalée dans le temps. Quand ils sont administrés séparément, le dérivé PBD de formule (II) peut être administré en premier ou en second, et respectivement la fludarabine en second ou en premier.

Une composition selon l'invention peut contenir des agents thérapeutiques autres que le dérivé PBD de formule (II) et la fludarabine. Ainsi, une composition selon la présente invention contient le dérivé PBD de formule (II), la fludarabine et optionnellement au moins un autre agent thérapeutique. De préférence, une composition selon la présente invention contient le dérivé PBD de formule (II) de formule (II), la fludarabine et un autre agent thérapeutique choisis parmi les anticorps monoclonaux. Comme exemple d'anticorps monoclonaux, on peut citer le rituximab (Rituxane^{®}) ou l'alemtuzumab (Campath^{®}).

Des compositions thérapeutiques telles que définies ci-dessus comprennent des quantités thérapeutiquement efficaces du dérivé PBD de formule (II) de formule (II) et de la fludarabine, et des supports pharmaceutiquement acceptables pour former ensemble ou de façon séparée une (ou des) composition(s) liquide(s) telles que solutions ou suspensions, ou une (ou des) composition(s) solide(s) telles que comprimés, poudres.

Indépendamment de l'administration envisagée, c'est à dire de façon simultanée, séparée ou étalée dans le temps, les deux composés : le dérivé PBD de formule (II) et la fludarabine, peuvent être administrés par des voies d'administration identiques ou différentes. Ils peuvent être administrés par des voies d'administration identiques ou différentes lorsqu'ils sont présents sous forme séparée, et par voie d'administration identiques lorsqu'ils sont présents sous forme non séparée. De préférence, le dérivé PBD de formule (II) est administré par voie intraveineuse. La fludarabine est administrée par les voies traditionnellement recommandées. Les autres agents additionnels peuvent être administrés par les voies d'administration recommandées dans le traitement des cancers.

De préférence, le dérivé PBD de formule (II) peut être utilisé à une concentration comprise entre 1.10⁻¹¹M et 1.10⁻⁷M. De préférence également, la fludarabine peut être utilisée à une concentration comprise entre 1.10⁻⁷M et 1.10⁻⁵M.

De préférence, le ratio molaire (dérivé PBD de formule (II) fludarabine) est inférieur à 1/250 et plus préférentiellement aux environs de 1/100 et en particulier de 1/100.

Le dérivé PBD de formule (II) peut être administré par voie intraveineuse toutes les trois semaines à une dose comprise entre 1 et 150 µg/m², et de préférence entre 10 et 100 µg/m².

L'activité d'une composition selon la présente invention peut être déterminée selon les deux protocoles suivants : *in vitro* sur les cellules B-CLL humaines, *in vivo* sur des modèles de leucémie, et par l'évaluation des lésions du DNA.

A moins qu'ils ne soient définis autrement, tous les termes techniques et scientifiques utilisés dans la présente demande ont la signification communément comprise par un spécialiste du domaine auquel appartient l'invention.

### PARTIE EXPÉRIMENTALE :

Les fourchettes de doses utilisées dans cette étude sont les suivantes :
fludarabine : 1.10⁻⁷M - 1.10⁻⁵M
dérivé PBD de formule (II) : 1.10⁻¹¹M - 1.10⁻⁷M

Dans le but d'établir la fourchette de dose appropriée pour chaque agent thérapeutique, on établit les courbes dose-réponse et les valeurs de DL₅₀ sont calculées en utilisant une méthode d'analyse de régression non-linéaire (sigmoïdale).

Dans les expériences, les cellules de leucémie lymphoïde chronique B sont traitées avec différentes dilutions pour chacun des produits pris individuellement ou combinés mais alors avec un ratio molaire fixe.

L'analyse des effets de la combinaison des 2 composés est faite en utilisant la méthode de Chou et Talalay (Chou TC, Talalay P., Quantitative analysis of dose-effect relationships : the combined effects of multiple drugs or enzyme inhibitors. Adv.Enzyme Regul. 1984 ; 22: 27-55). Cette méthode utilise les courbes dose-réponse pour chacun des composés et pour un ratio fixe des 2 composés en faisant varier leur concentration. Puis l'index de combinaison (IC) est calculé. Une valeur de IC inférieure à 1 indique une synergie, et une valeur de 1 ou supérieure à 1 suggère un effet additif ou un antagonisme respectivement.

Trois ratios molaires (dérivé PBD/fludarabine) sont ainsi définis : 1/100, 1/250 et 1/500. La valeur de l'index de combinaison obtenu pour chacun de ces ratios est la suivante :

| | Ratio molaire | | |
|---|---|---|---|
| | 1/100 | 1/250 | 1/500 |
| IC | 0,41 | 0,93 | 1,42 |

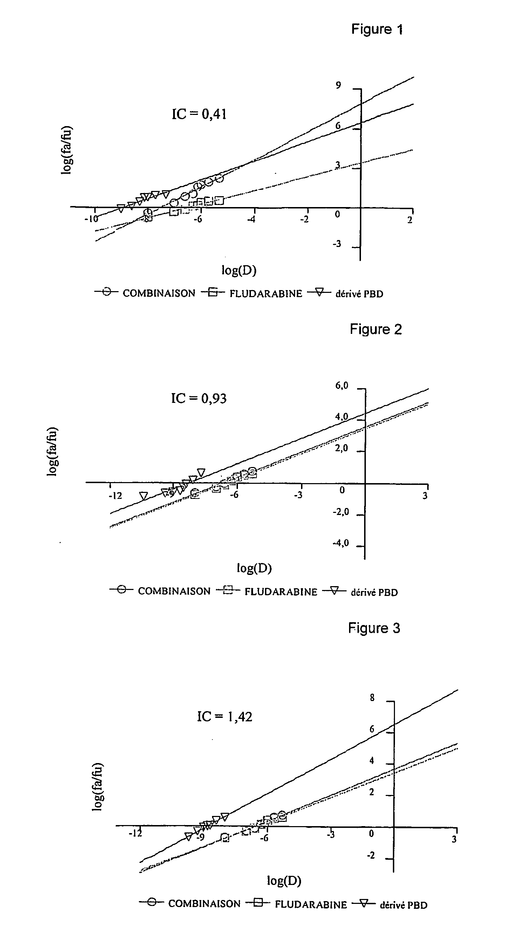

Les courbes de l'effet dose-réponse pour les composés pris individuellement (dérivé PBD et fludarabine) ou en combinaison, avec un ratio molaire fixe de 1/100, 1/250 et 1/500, sont représentées par les figures 1-3 respectivement (fa = fraction affectée (apoptose) ; fu = fraction non affectée (viable), log D = log de la concentration du produit).

On peut ainsi noter que le ratio molaire (dérivé PBD de formule (II) : fludarabine) (1/100) donne une valeur de l'index de combinaison de 0,41 ce qui montre qu'avec un tel ratio, la combinaison présente un effet synergique par opposition au ratio de 1/500 qui suggère plutôt un effet antagoniste.

## Revendications

1. Utilisation d'une composition thérapeutique d'un dérivé PBD de formule (II) 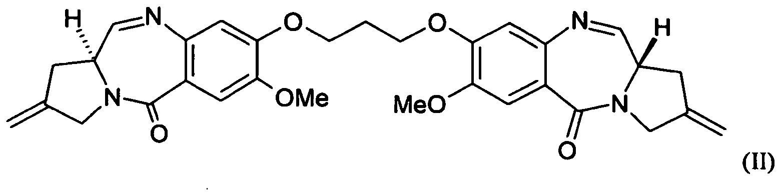 et/ou un mono- et/ou bis-carbinol formé par addition d'eau ou d'alcool,
et/ou un sel d'addition avec un acide et/ou une forme solvatée et/ou une forme hydratée,
et/ou un sel d'addition avec un acide et/ou une forme solvatée et/ou une forme hydratée d'un tel composé, sous forme amorphe ou crystalline, sous forme racémique ou sous une forme optiquement active,
et/ou tout mélange de quelques composés ou de tous les composés listés ci-dessus,
en combinaison avec la fludarabine, pour la préparation d'un médicament pour le traitement du cancer.

2. Utilisation selon la revendication 1, pour le traitement de maladies hématologiques.

3. Utilisation selon l'une des revendications précédentes, pour le traitement de leucémies.

4. Utilisation selon la revendication 3, pour le traitement de la leucémie lymphoïde chronique.

5. Utilisation selon l'une des revendications précédentes, **caractérisée en ce que** le dérivé PBD est pour utilisation à une concentration comprise entre 1.10⁻¹¹M et 1.10⁻⁷M.

6. Utilisation selon l'une des revendications précédentes, **caractérisée en ce que** la fludarabine est utilisation dans une concentration comprise entre 1.10⁻⁷M et 1.10⁻⁵M.

7. Utilisation selon l'une des revendications précédentes, **caractérisée en ce que** le ratio molaire (dérivé PBD/fludarabine) est inférieur à 1/250.

8. Utilisation selon l'une des revendications précédentes, **caractérisée en ce que** le ratio molaire (dérivé PBD/fludarabine) est aux environs de 1/100.

9. Utilisation selon l'une des revendications précédentes, **caractérisée en ce que** le ratio molaire (dérivé PBD/fludarabine) est de 1/100.

10. Produit comprenant le dérivé PBD de formule (II) 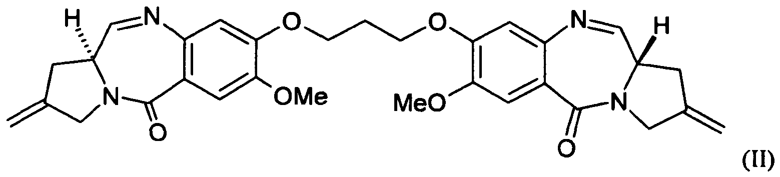 et/ou un mono- et/ou bis-carbinol formé par addition d'eau ou d'alcool,
et/ou un sel d'addition avec un acide et/ou une forme solvatée et/ou une forme hydratée,
et/ou un sel d'addition avec un acide et/ou une forme solvatée et/ou une forme hydratée d'un tel composé, sous forme amorphe ou crystalline, sous forme racémique ou sous une forme optiquement active,
et/ou tout mélange de quelques composés ou de tous les composés listés ci-dessus,
et la fludarabine, en tant que produit de combinaison pour une utilisation simultanée, séparée ou étalée dans le temps, pour utilisation dans le traitement du cancer.

11. Produit selon la revendication 10, en tant que produit de combinaison pour une utilisation simultanée.

12. Produit selon la revendication 10, en tant que produit de combinaison pour une utilisation séparée.

13. Produit selon la revendication 10, en tant que produit de combinaison pour une utilisation étalée dans le temps.

14. Produit selon l'une des revendications 10 à 13, pour le traitement de maladies hématologiques.

15. Produit selon l'une des revendications 10 à 14, pour utilisation dans le traitement des leucémies.

16. Produit selon la revendication 15, pour utilisation dans le traitement de la leucémie lymphoïde chronique.

17. Produit selon l'une des revendications 10 à 16, **caractérisé en ce que** le dérivé PBD est pour utilisation à une concentration comprise entre 1.10⁻¹¹M et 1.10⁻⁷M.

18. Produit selon l'une des revendications 10 à 17, **caractérisé en ce que** la fludarabine est pour utilisation à une concentration comprise entre 1.10⁻⁷M et 1.10⁻⁵M.

19. Produit selon l'une des revendications 10 à 18, **caractérisé en ce que** le ratio molaire (dérivé PBD/fludarabine) est de 1/250.

20. Produit selon l'une des revendications 10 à 18, **caractérisé en ce que** le ratio molaire (dérivé PBD/fludarabine) est aux environs de 1/100.

21. Produit selon l'une des revendications 10 à 18, **caractérisé en ce que** le ratio molaire (dérivé PBD/fludarabine) est de 1/100.

## Claims

1. Use of a therapeutic composition of a PBD derivative of formula (II) 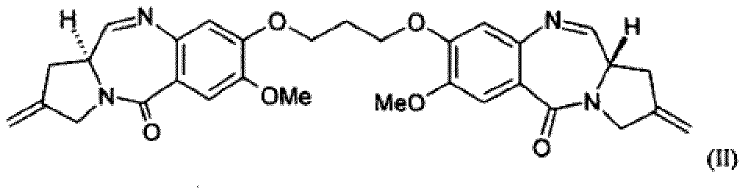 and/or a mono- and/or bis-carbinol formed by the addition of water of alcohol,
and/or an addition salt with an acid and/or a solvated form and/or a hydrated form. and/or an addition salt with an acid and/or a solvated form and/or a hydrated form of such a compound in an amorphous or crystalline form, in racemic form or in an optically active form,
and/or any mixture of some of the compounds or all of the compounds listed above, in combination with fludarabine, for the preparation of a medicament for the treatment of cancer.

2. The use according to claim 1, for the treatment of haematological diseases.

3. The use according to claim 1, for the treatment of leukaemia.

4. The use according to claim 3, for the treatment of chronic lymphoid leukaemia.

5. The use according to one of the preceding claims, **characterised in that** the PBD derivative is for use at a concentration between 1.10⁻¹¹M and 1.10⁻⁷M.

6. The use according to one of the preceding claims, **characterised in that** the fludarabine is used at a concentration between 1.10⁻⁷M and 1.10⁻⁵M.

7. The use according to one of the preceding claims, **characterised in that** the molar ratio (PBD derivative/fludarabine) is less than 1/250.

8. The use according to one of the preceding claims, **characterised in that** the molar ratio (PBD derivative/fludarabine) is approximately 1/100.

9. The use according to one of the preceding claims, **characterised in that** the molar ratio (PBD derivative/fludarabine) is 1/100.

10. Product comprising a PBD derivative of formula (II) 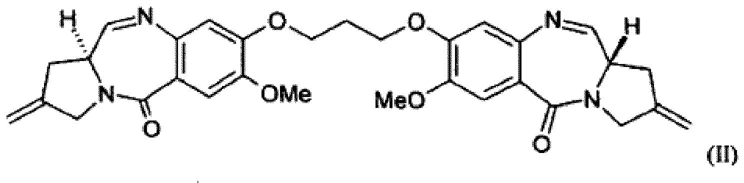 and/or a mono- and/or bis-carbinol formed by the addition of water of alcohol, and/or an addition salt with an acid and/or a solvated form and/or a hydrated form. and/or an addition salt with an acid and/or a solvated form and/or a hydrated form of such a compound in an amorphous or crystalline form, in racemic form or in an optically active form,
and/or any mixture of some of the compounds or all of the compounds listed above, and fludarabine, as a combination product for use simultaneously, separately or spread over time, for the treatment of cancer.

11. The product according to claim 10, as a combination product for use simultaneously.

12. The product according to claim 10, as a combination product for use separately.

13. The product according to claim 10, as a combination product for use spread over time.

14. The product according to one of claims 10 to 13, for the treatment of hematological diseases.

15. The product according to one of claims 10 to 14, for use in the treatment of leukaemias.

16. The product according to claim 15, for use in the treatment of chronic lymphoid leukaemia.

17. The product according to one of claims 10 to 16, **characterised in that** the PBD derivative is for use at a concentration between 1.10⁻¹¹M and 1.10⁻⁷M.

18. The product according to one of claims 10 to 17, **characterised in that** the fludarabine is for use at a concentration between 1.10⁻⁷M and 1.10⁻⁵M.

19. The product according to one of claims 10 to 18, **characterised in that** the molar ratio (PBD derivative/fludarabine) is less than 1/250.

20. The product according to one of claims 10 to 18, **characterised in that** the molar ratio (PBD derivative/fludarabine) is approximately 1/100.

21. The product according to one of claims 10 to 18, **characterised in that** the molar ratio (PBD derivative/fludarabine) is 1/100.

## Patentansprüche

1. Verwendung einer therapeutischen Zusammensetzung eines PBD-Derivats der Formel (II) 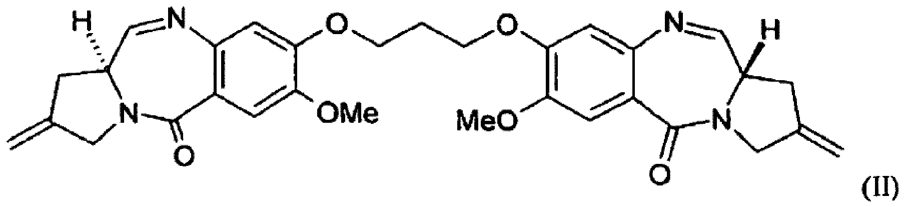 und/oder eines durch Addition von Wasser oder Alkohol gebildeten Mono- und/oder Biscarbinols,
und/oder eines Säureadditionssalzes und/oder einer solvatisierten Form und/oder einer hydrierten Form,
und/oder eines Säureadditionssalzes und/oder einer solvatisierten Form und/oder einer hydrierten Form einer solchen Verbindung in amorpher oder kristalliner Form, in racemischer oder optisch aktiver Form,
und/oder eines beliebigen Gemischs mancher oder aller der oben angeführten Verbindungen,
in Kombination mit Fludarabin zur Herstellung eines Medikaments zur Behandlung von Krebs.

2. Verwendung nach Anspruch 1 zur Behandlung von hämatologischen Erkrankungen.

3. Verwendung nach einem der vorangegangenen Ansprüche zur Behandlung von Leukämieerkrankungen.

4. Verwendung nach Anspruch 3 zur Behandlung von chronischer lymphatischer Leukämie.

5. Verwendung nach einem der vorangegangenen Ansprüche, **dadurch gekennzeichnet, dass** das PBD-Derivat zur Verwendung in einer Konzentration zwischen 1-10⁻¹¹M und 1,10⁻⁷M dient.

6. Verwendung nach einem der vorangegangenen Ansprüche, **dadurch gekennzeichnet, dass** das Fludarabin zur Verwendung in einer Konzentration zwischen 1.10⁻⁷ Mund 1,10⁻⁵ M dient.

7. Verwendung nach einem der vorangegangenen Ansprüche, **dadurch gekennzeichnet, dass** das Molverhältnis (PBD-Derivat/Fludarabin) weniger als 1/250 beträgt.

8. Verwendung nach einem der vorangegangenen Ansprüche, **dadurch gekennzeichnet, dass** das Molverhältnis (PBD-Derivat/Fludarabin) etwa 1/100 beträgt.

9. Verwendung nach einem der vorangegangenen Ansprüche, **dadurch gekennzeichnet, dass** das Molverhältnis (PBD-Derivat/Fludarabin) 1/100 beträgt.

10. Produkt, das ein PBD-Derivat der Formel (II) 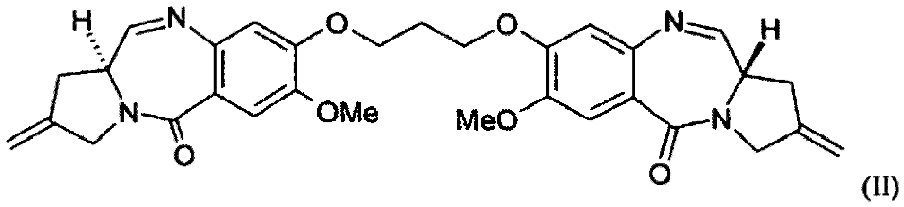 umfasst,
und/oder ein durch Addition von Wasser oder Alkohol gebildetes Mono- und/oder Biscarbinol,
und/oder ein Säureadditionssalz und/oder eine solvatisierte und/oder hydrierte Form, und/oder ein Säureadditionssalz und/oder eine solvatisierte und/oder eine hydrierte Form einer solchen Verbindung in amorpher oder kristalliner Form, in racemischer oder optisch aktiver Form,
und/oder ein beliebiges Gemisch mancher oder aller der oben angeführten Verbindungen,
sowie Fludarabin als Kombinationsprodukt für eine gleichzeitige, getrennte oder zeitlich versetzte Anwendung zur Verwendung bei der Behandlung von Krebs.

11. Produkt nach Anspruch 10 als Kombinationsprodukt zur gleichzeitigen Anwendung.

12. Produkt nach Anspruch 10 als Kombinationsprodukt zur getrennten Anwendung.

13. Produkt nach Anspruch 10 als Kombinationsprodukt zur zeitlich versetzten Anwendung.

14. Produkt nach einem der Ansprüche 10 bis 13 zur Behandlung von hämotologischen Erkrankungen.

15. Produkt nach einem der Ansprüche 10 bis 14 zur Verwendung zur Behandlung von Leukämieerkrankungen.

16. Produkt nach Anspruch 15 zur Verwendung zur Behandlung von chronischer lymphatischer Leukämie.

17. Produkt nach einem der Ansprüche 10 bis 16, **dadurch gekennzeichnet, dass** das PBD-Derivat zur Verwendung in einer Konzentration zwischen 1-10⁻¹¹M und 1,10⁻⁷M dient.

18. Produkt nach einem der Ansprüche 10 bis 17, **dadurch gekennzeichnet, dass** das Fludarabin zur Verwendung in einer Konzentration zwischen 1.10⁻⁷M und 1,10⁻⁵ M dient.

19. Produkt nach einem der Ansprüche 10 bis 18, **dadurch gekennzeichnet, dass** das Molverhältnis (PBD-Derivat/Fludarabin) weniger als 1/250 beträgt.

20. Produkt nach einem der Ansprüche 10 bis 18, **dadurch gekennzeichnet, dass** das Molverhältnis (PBD-Derivat/Fludarabin) etwa 1/100 beträgt.

21. Produkt nach einem der Ansprüche 10 bis 18, **dadurch gekennzeichnet, dass** das Molverhältnis (PBD-Derivat/Fludarabin) 1/100 beträgt.
